# EUROPÄISCHE PATENTANMELDUNG

(11) **EP 4 420 653 A1**
(43) Veröffentlichungstag der Anmeldung: **28.08.2024**
(21) Anmeldenummer: 23209328.6
(22) Anmeldetag: 13.11.2023
(51) Int. Cl.: A61K 8/34, A61K 8/92, A61K 8/9789, A61Q 17/04, A61Q 19/08, A61K 8/06, A61K 8/31, A61K 8/37, A61K 8/67, A61K 8/86, A61Q 19/00, A61K 8/73

(54) **HAUTFEUCHTIGKEITSSPENDENDE KÖRPERMILCH**

(30) Priorität: 21.02.2023 DE 102023201528
(71) Anmelder: Beiersdorf AG, 22529 Hamburg (DE)
(72) Erfinder: Bleckmann, Andreas, 22926 Ahrensburg (DE); Sellckau, Sabine, 22455 Hamburg (DE); Wischhöfer, Svea, 21109 Hamburg (DE)
(74) Vertreter: Beiersdorf AG

(57) **Zusammenfassung**

Eine neuartige Körpermilch zur verbesserten Hautfeuchtigkeit umfasst Hyaluronsäure und/oder deren Salze, PEG-40 Sorbitan Perisostearat und Polyglyceryl-3 Diisostearat und Tocopherol, sowie ein oder mehrere Lipide gewählt aus der Gruppe Isopropylpalmitat, Isododekan, Mandelöl (Prunus Amygdalus Dulcis Oil), wobei die Zubereitung frei von BHT ist.

## Beschreibung

Die vorliegende Erfindung betrifft eine kosmetische oder dermatologische Zubereitung enthaltend Hyaluronsäure und/oder deren Salze, Polyglyceryl-3 Diisostearat, PEG-40 Sorbitan Perisostearat und Tocopherol, sowie ein oder mehrere Lipide gewählt aus der Gruppe Isopropylpalmitat, Isododekan und Mandelöl (Prunus Amygdalus Dulcis Oil).

Äußere Einflüsse, insbesondere Umwelteinflüsse, Sonnenbestrahlung und Hautreinigung führen dazu, dass die Haufeuchtigkeitsbalance gestört wird.

Damit die Haut ihre biologischen Funktionen im vollen Umfang erfüllen kann, bedarf sie der regelmäßigen Reinigung und Pflege. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Zur Pflege der Haut werden den Verbrauchern heutzutage eine Vielzahl von kosmetischen Zubereitungen, meist in Form von Cremes und Lotionen, d.h. als Emulsion, angeboten. Produkte, welche die Alterungserscheinungen der Haut, negative Umwelteinflüsse und eben Hauterkrankungen temporär oder dauerhaft verzögern oder beseitigen, haben dabei eine stetig wachsende Bedeutung. Neben Wasser zur Hautbefeuchtung sowie Ölen und Lipiden zur Rückfettung der Haut enthalten derartige Hautpflegeprodukte eine Vielzahl an Wirk- Hilfs- und Zusatzstoffen.

Hyaluronsäure (auch nach Nomenklatur Hyaluronan, Abkürzung HA, CAS: 9004-61-9) ist ein Glykosaminoglykan, das einen wichtigen Bestandteil des Bindegewebes darstellt. Die HA ist durch eine Disaccharid-Wiederholungseinheit gemäß nachstehender Struktur gekennzeichnet.

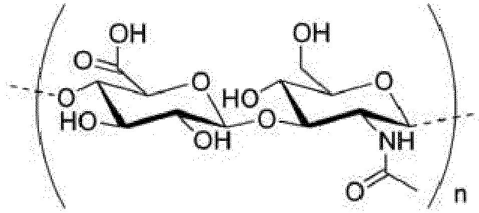

HA ist ein lineares, saures Polysaccharid, welches aus einer Vielzahl alternierender Dissacharideinheiten aus 1 ,3-glykosidisch verknüpfter N-Acetyl-β-D-Glucosamin- (GlcNAc) und β-D-Glucoronsäure-Moleküle (GIcA) besteht. Jede der disaccharidischen Grundeinheiten ist durch eine ß(1-4)-Bindung mit der nächsten verknüpft. Die Anzahl der Grundeinheiten kann mehr als 10.000 mit einer Molmasse von 4 Mio. Dalton erreichen. Aufgrund ihrer hydrophilen Säuregruppen und den Hydroxygruppen besitzt HA die Fähigkeit eine Vielzahl von Wasserstoffbrückenbindungen auszubilden, über die Hydrationen angelagert werden können und sie somit mit Wasser homogen mischbar ist. In wässriger Lösung ist HA in der Lage unter Erweiterung des durchschnittlichen Kettenabstandes immer mehr Wasser zu binden, da die negativen Ladungen eine Abstoßung der nahe beieinander liegenden Kettenabschnitte bewirken. Unter physiologischen Bedingungen liegen die Carboxygruppen der HA praktisch vollkommen dissoziiert vor und stellen eine polyanionische Verbindung dar. Im Allgemeinen wird von Hyaluronsäure gesprochen, unabhängig davon, ob HA selbst oder die Salze gemeint sind. Aufgrund der viskoelastischen Eigenschaften und ihrer Biokompatibilität hat HA eine therapeutische Bedeutung als Injektionslösung in der Ophthalmologie, Orthopädie und der kosmetischen Chirurgie.

Hyaluronsäure ist ein natürlicher Bestandteil der Haut. In Kosmetikprodukten werden zwei Formen der Hyaluronsäure verwendet, eine kurzkettige und eine langkettige Form. Die kurzkettige Hyaluronsäure durchdringt die obere Hautschicht und beeinflusst die innere Feuchtigkeitsbalance. Die langkettige Hyaluronsäure soll hingegen einen aufpolsternden Effekt auf der Haut zeigen. Sie bindet effektiv Feuchtigkeit in der oberen Hautschicht. Ihre feuchtigkeitsbindende Wirkung ergibt sich daraus, dass sie ihren Hydratmantel erst nach und nach abgibt und auf diese Weise über einen langen Zeitraum auf der Haut wirkt. Weitere Aufgaben der HA sind die Versorgung von Bindegewebe und Haut mit Feuchtigkeit und Nährstoffen.

Das Natriumsalz der Hyaluronsäure wird u.a. auch als Moisturizer (Feuchthaltemittel) für die Herstellung kosmetischer Mittel verwendet (Römpp online Lexikon Version 2.5, 2004).

Hyaluronsäure ist im vernetzten Zustand handelsüblich erhältlich (z. B. Hylaform@, eine vernetzte Hyaluronsäure der Fa. Biomatrix, NJ, USA ; zur Herstellung vgl. auch US-A-4 713 448, US-A-4 605 691, APC@ der Fa. Fidia, Incert@ der Fa. Anika Therapeutics, interge) der Fa. LifeCore oder Restylane@ der Fa. Q-Med).

In jüngerer Zeit gibt es einen verstärkten Trend hin zu "natürlichen" Kosmetika, deren Inhaltsstoffe möglichst nicht mehr aus Erdölprodukten stammen oder chemisch synthetisiert sein sollen. Dieser Trend überlappt sich heutzutage mit dem Trend hin zu "veganen" Produkten. Dabei stellt die Suche nach alternativen Inhaltsstoffen, welche diese Kriterien erfüllen, die Produktentwickler vor besondere Herausforderungen. Denn der Austausch der bekannten Inhaltstoffe wie Mineralöle, Silikonöle, Polyacrylate wird praktisch immer mit Nachteilen im Hinblick auf die Produkteigenschaften erkauft. Die Zubereitungen werden instabil und sensorisch unattraktiv, was sich beispielsweise beim Verteilen der Zubereitung auf der Haut und dem mangelnden Einzugsvermögen unangenehm bemerkbar macht. Diese Probleme treten insbesondere bei Wasser-in-Öl-Emulsionen (W/O-Emulsionen) auf, die sich in jüngerer Zeit wieder einer wachsenden Beliebtheit erfreuen. So neigen W/O-Emulsionen ohne Mineralöle und Mineralwachse sowie ohne Polyethylenglycolethern oder -estern (so genannten PEG-Derivaten) zu einer verstärkten Instabilität. Bei längerer Lagerungsdauer kommt es insbesondere bei höheren Temperaturen schnell zu Phasentrennungen in Form von Öl- und/oder Wasserabscheidungen, die sich auch nicht ohne weiteres durch einen höheren Emulgatorgehalt kompensieren lassen.

Es war daher die Aufgabe der vorliegenden Erfindung, eine kosmetische oder dermatologische Zubereitung zur Verfügung zu stellen, die möglichst auf BHT, Polyethylenglycolethern und -estern verzichtet sowie vorteilhaft frei von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin ist.

Gelöst werden die Aufgaben durch eine kosmetische oder dermatologische Zubereitung umfassend Hyaluronsäure und/oder deren Salze, Polyglyceryl-3 Diisostearat, PEG-40 Sorbitan Perisostearat und Tocopherol, sowie ein oder mehrere Lipide gewählt aus der Gruppe Isopropylpalmitat, Isododekan und Mandelöl (Prunus Amygdalus Dulcis Oil).

Die erfindungsgemäße Zubereitung wird vorteilhafterweise als Wasser-in-Öl (W/O)-Emulsionen bereitgestellt und ist eine hautfeuchtigkeitsspendende Körpermilch
Idealerweise sind die erfindungsgemäßen Zubereitungen auch frei von BHT, Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, ausgenommen die erfindungsgemäß enthaltenen Stoffe, wie PEG-40 Sorbitan Perisostearate.

Frei von bedeutet, dass deren Anteil unter 0,1 Gew.% liegt, falls durch Einschleppungen oder Verunreinigungen der ein oder andere Stoff dennoch enthalten sein könnte.

Zwar kennt der Fachmann die DE 102017221672 und DE 102018217130, doch konnten diese Schriften nicht den Weg zur vorliegenden Erfindung weisen.

Hyaluronsäure und/oder deren Salze werden bevorzugt zu einem Anteil von 0,0005 bis 5 Gew.%, bevorzugt im Bereich von 0,001 bis 1 Gew.%, bezogen auf die Gesamtmasse der Zubereitung, zugesetzt.

Die Zubereitung umfasst vorteilhaft Polyglyceryl-3 Diisostearat zu einem Anteil von 0,1 bis 3,0 Gew.%, besonders bevorzugt ist ein Anteil im Bereich von 0,5 bis 2 Gew.%. bezogen auf das Gesamtgewicht der Zubereitung.

Die Zubereitung umfasst vorteilhaft PEG-40 Sorbitan Perisostearat zu einem Anteil von 0,1 bis 3,0 Gew.%, besonders bevorzugt ist ein Anteil im Bereich von 0,5 bis 2 Gew.%. bezogen auf das Gesamtgewicht der Zubereitung.

Vorteilhaft werden PEG-40 Sorbitan Perisostearat und Polyglyceryl-3 Diisostearat in einem Gewichtsverhältnis im Bereich von 3:1 bis 1:2, insbesondere im Verhältnis 2:1 bis 1:1.

Tocopherol ist vorteilhaft zu einem Anteil von 0,01 bis 2,0 Gew.%, besonders bevorzugt ist ein Anteil im Bereich von 0,05 bis 1,5 Gew.%. bezogen auf das Gesamtgewicht der Zubereitung, enthalten.

Es ist vorteilhaft wenn ein oder mehrere, bevorzugt alle Lipide gewählt aus der Gruppe Isopropylpalmitat, Isododekan, Mandelöl (Prunus Amygdalus Dulcis Oil) enthalten sind zu einem Anteil im Bereich von jeweils 0,1 bis 15 Gew.%, bezogen auf die Die Gesamtmasse der Zubereitung.

Enthält die Zubereitung Isopropylpalmitat, so wird diese Komponente erfindungsgemäß vorteilhaft zu einem Anteil von 1,0 bis 15,0 Gew.%, bezogen auf das Gesamtgewicht der Zubereitung eingesetzt. Besonders bevorzugt sind 2 bis 12 Gew.%.

Vorteilhafte Ausführungsformen der vorliegenden Erfindung sind ferner dadurch gekennzeichnet, dass die Zubereitung ein oder mehrere Pflanzenöle enthält.

Dabei ist es erfindungsgemäß bevorzugt, wenn die Zubereitung Mandelöl (INCI: Prunus Amygdalus Dulcis Oil) enthält.

Enthält die Zubereitung Mandelöl (INCI: Prunus Amygdalus Dulcis Oil), so wird diese Komponente erfindungsgemäß vorteilhaft in einer Konzentration von 0,1 bis 0,5 Gew.%, bezogen auf das Gesamtgewicht der Zubereitung eingesetzt.

Vorteilhaft umfasst die erfindungsgemäße Zubereitung ein oder mehrere weitere Hautbefeuchtungsmittel, insbesondere Glycerin. Diese sind jeweils bevorzugt zu einem Anteil von 2 bis 15 Gew.%, insbesondere im Bereich von 3 bis 10 Gew.%, vorteilhaft im Bereich von 4 bis 8 Gew.%, jeweils bezogen auf die Gesamtmasse der Zubereitung.

Als Befeuchtungsmittel, auch als Moisturizer bezeichnet, werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL)) genannt, zu reduzieren und/oder die Hydratation der Hornschicht positiv zu beeinflussen.

Vorteilhafte Moisturizer im Sinne der vorliegenden Erfindung sind beispielsweise Glycerin, Butylenglykol, Propylencarbonat. Weitere Befeuchtungsmittel sind beispielsweise polymere Moisturizer aus der Gruppe der wasserlöslichen und/oder in Wasser quellbaren und/oder mit Hilfe von Wasser gelierbaren Polysaccharide.

Überraschenderweise zeigte sich die Kombination aus ein oder mehreren erfindungsgemäßen Lipiden, Polyglyceryl-3 Diisostearat, PEG-40 Sorbitan Perisostearat und Hyaluronsäure bzw. dessen Salz, sowie gegebenenfalls Glycerin, als ein die Hautfeuchtigkeit fördernder Komplex, der erfindungsgemäß Moisture-Komplex genannt wird.

Das Kapazitätsmessprinzip des Corneometer^{®} wird weltweit angewendet und dient zur Bestimmung der Hautfeuchtigkeit. Corneometerwerte können daher zur Beurteilung der Hautfeuchtebalance genutzt werden und ermittelte Corneometerdaten geben einen Hinweis auf den Feuchtigkeitsgehalt der Haut.

Die erfindungsgemäße Kombination, der die Hautfeuchtigkeit fördernde Komplex, zeigt gegenüber dem unbehandelten Hautareal und gegenüber dem nur mit jeweils einem Bestandteil des Komplexes behandelten Hautareals jeweils höhere und damit verbesserte Corneometerwerte.

Der erfindungsgemäß Moisture-Komplex fördert die Hautfeuchtigkeit und beeinflusst positiv die Hydratation der Haut-Hornschicht.

Die erfindungsgemäße Zubereitung kann darüber hinaus weitere für Kosmetika übliche Inhaltsstoffe enthalten.

### Beispiel

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCI | Gew.% | Gew. % | Gew. % |
|---|---|---|---|
| Tocopherol | 0,05 | 0,5 | 0,6 |
| Sodium Hyaluronate | 0,001 | 0,0015 | 0,002 |
| Cera Microcristallina + Paraffinum Liquidum + Tocopheryl Acetate + BHT | 3 | 2,5 | 4 |
| Paraffinum Liquidum | 9 | 9,5 | 8 |
| Isopropyl Palmitate | 3 | 3,5 | 2,5 |
| Isododecane | 4,5 | 4 | 5 |
| Prunus Amygdalus Dulcis Oil | 0,2 | 0,3 | 0,1 |
| PEG-40 Sorbitan Perisostearate | 1,77 | 1,9 | 2 |
| Polyglyceryl-3 Diisostearate + Aqua | 1,43 | 1,5 | 1,6 |
| Parfum | 0,35 | 0,3 | 0,4 |
| Glycerin + Aqua | 5,3 | 6 | 7 |
| Citric Acid | 0,086 | 0,09 | 0,08 |
| Sodium Citrate + Aqua | 0,174 | 0,18 | 0,17 |
| Potassium Sorbate | 0,125 | 0,13 | 0,1 |
| Magnesium Sulfate | 0,7 | 0,8 | 0,6 |
| Aqua | Ad 100 | Ad 100 | Ad 100 |

## Patentansprüche

1. Kosmetische oder dermatologische Zubereitung umfassend Hyaluronsäure und/oder deren Salze, Polyglyceryl-3 Diisostearat, PEG-40 Sorbitan Perisostearat und Tocopherol, sowie ein oder mehrere Lipide gewählt aus der Gruppe Gruppe Isopropylpalmitat, Isododekan und Mandelöl (Prunus Amygdalus Dulcis Oil).

2. Zubereitung nach Anspruch 1 **dadurch gekennzeichnet, dass** die Zubereitung eine Wasser-in-Öl-Emulsion (W/O-Emulsion) ist.

3. Zubereitung nach Anspruch 1 oder 2 **dadurch gekennzeichnet, dass** die Zubereitung frei ist von BHT, Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, ausgenommen PEG-40 Sorbitan Perisostearat.

4. Zubereitung nach einem der vorstehenden Ansprüche **dadurch gekennzeichnet, dass** die Zubereitung Hyaluronsäure und/oder deren Salze zu einem Anteil von 0,0005 bis 5 Gew.%, bevorzugt im Bereich von 0,001 bis 1 Gew.%, bezogen auf die Gesamtmasse der Zubereitung, enthält.

5. Zubereitung nach einem der vorstehenden Ansprüche **dadurch gekennzeichnet, dass** die Zubereitung Polyglyceryl-3 Diisostearat zu einem Anteil von 0,1 bis 3,0 Gew.%, besonders bevorzugt im Bereich von 0,5 bis 2 Gew.%, bezogen auf das Gesamtgewicht der Zubereitung, enthält.

6. Zubereitung nach einem der vorstehenden Ansprüche **dadurch gekennzeichnet, dass** die Zubereitung PEG-40 Sorbitan Perisostearat zu einem Anteil von 0,1 bis 3,0 Gew.%, besonders bevorzugt im Bereich von 0,5 bis 2 Gew.%. bezogen auf das Gesamtgewicht der Zubereitung, enthält.

7. Zubereitung nach einem der vorstehenden Ansprüche **dadurch gekennzeichnet, dass** die Zubereitung PEG-40 Sorbitan Perisostearat und Polyglyceryl-3 Diisostearat in einem Gewichtsverhältnis im Bereich von 3:1 bis 1:2, insbesondere im Verhältnis 2:1 bis 1:1, enthält.

8. Zubereitung nach einem der vorstehenden Ansprüche **dadurch gekennzeichnet, dass** die Zubereitung Tocopherol zu einem Anteil von 0,01 bis 2,0 Gew.%, besonders bevorzugt im Bereich von 0,05 bis 1,5 Gew.%. bezogen auf das Gesamtgewicht der Zubereitung, enthält.

9. Zubereitung nach einem der vorstehenden Ansprüche **dadurch gekennzeichnet, dass** die Zubereitung ein oder mehrere, bevorzugt alle Lipide gewählt aus der Gruppe Isopropylpalmitat, Isododekan, Mandelöl (Prunus Amygdalus Dulcis Oil) enthält zu einem Anteil im Bereich von jeweils 0,1 bis 15 Gew.%, bezogen auf die Die Gesamtmasse der Zubereitung.

10. Zubereitung nach einem der vorstehenden Ansprüche **dadurch gekennzeichnet, dass** die Zubereitung ein oder mehrere weitere Hautbefeuchtungsmittel, insbesondere Glycerin, umfasst.

11. Zubereitung nach Anspruch 10 **dadurch gekennzeichnet, dass** das oder die weiteren Hautbefeuchtungsmittel, insbesondere Glycerin, zu einem Anteil von 2 bis 15 Gew.%, insbesondere im Bereich von 3 bis 10 Gew.%, vorteilhaft im Bereich von 4 bis 8 Gew.%, jeweils bezogen auf die Gesamtmasse der Zubereitung, enthalten sind.

12. Verwendung der Kombination bestehend aus PEG-40 Sorbitan Perisostearat, Polyglyceryl-3 Diisostearat, Hyaluronsäure bzw. dessen Salz, ein oder mehreren Lipiden gewählt aus der Gruppe Isopropylpalmitat, Isododekan, Mandelöl (Prunus Amygdalus Dulcis Oil) und gegebenenfalls Glycerin, in kosmetischen oder dermatologischen Zubereitungen als ein die Hautfeuchtigkeit fördernden Komplex.
